# EUROPEAN PATENT SPECIFICATION

(11) **EP 4 275 741 B1**
(45) Date of publication and mention of the grant of the patent: **11.09.2024**
(21) Application number: 22172682.1
(22) Date of filing: 11.05.2022
(51) Int. Cl.: A61N 5/10

(54) **COMPUTER IMPLEMENTED METHOD FOR REDUCING THE RISK OF INTERRUPTING AN IRRADIATION TREATMENT SESSION DUE TO A DEVIATION FROM A PLANNED VALUE OF AN OPERATING PARAMETER OF A PARTICLE ACCELERATING SYSTEM**
COMPUTERIMPLEMENTIERTES VERFAHREN ZUR VERRINGERUNG DES RISIKOS DER UNTERBRECHUNG EINER BESTRAHLUNGSBEHANDLUNG AUFGRUND EINER ABWEICHUNG VON EINEM GEPLANTEN WERT EINES BETRIEBSPARAMETERS EINES TEILCHENBESCHLEUNIGUNGSSYSTEMS
PROCÉDÉ MIS EN UVRE PAR ORDINATEUR POUR RÉDUIRE LE RISQUE D'INTERRUPTION D'UNE SÉANCE DE TRAITEMENT PAR IRRADIATION EN RAISON D'UN ÉCART PAR RAPPORT À UNE VALEUR PLANIFIÉE D'UN PARAMÈTRE DE FONCTIONNEMENT D'UN SYSTÈME D'ACCÉLÉRATION DE PARTICULES

(43) Date of publication of application: 15.11.2023
(73) Proprietor: Ion Beam Applications, 1348 Louvain-la-Neuve (BE)
(72) Inventor: LABARBE, Rudi, 1348 Louvain-la-Neuve (BE); HOTOIU, Lucian, 1348 Louvain-la-Neuve (BE); PIN, Arnaud, 1348 Louvain-la-Neuve (BE)
(74) Representative: Connor, Marco Tom

(56) References cited:
- WO-A1-2011/162021
- US-A1- 2020 353 288

## Description

### TECHNICAL FIELD

The present invention is in the general field of treatment of tumoral cells by irradiation with accelerated particles, such as protons. In particular, it concerns a method for setting the operating parameters of a given particle accelerating system ensuring that the given particle accelerating system can deliver within a predefined confidence level beamlets of accelerated particles fulfilling the requirements of a treatment plan (TP) comprised within an acceptable band of variation (BV). This method is advantageous in that it reduces to within the predefined confidence level the risk of having to suddenly interrupt a patient irradiation session because the given particle accelerating system failed to deliver one or more beamlets satisfying the TP within the acceptable band of variation.

### BACKGROUND OF THE INVENTION

Radiation therapy with particles or waves, such as protons beams, electron beams, heavy ions beams, x-rays, *γ*-rays, and the like, has become an essential tool for treating patients with tumours. Methods of planning radiation therapy are disclosed i. a. in document US 2020/0353288 A1.

Pencil beam scanning (PBS) is a technique consisting of steering beamlets of charged particles towards a target comprising tumoral cells defining a structure of interest. PBS reduces unnecessary radiation exposure to surrounding non-cancerous cells by shaping the area being treated to mirror the tumour geometry of the structure of interest. Pencil beam scanning can treat a tumour with a single beam composed of various beamlets or with multiple beams of different orientations each composed of various beamlets, sometimes called intensity modulated proton therapy (IMPT). Beside the geometry of the target, PBS allows local tuning of the parameters of the beamlets depending on the position within the target. The parameters can include the position and the monitor unit of each beamlet as well as the scanning sequence of the beamlets, with starting time and end time of each beamlet.

Since both tumoural cells and healthy cells are damaged by such radiations, a major challenge in cancer treatment is to define a treatment plan (TP) ensuring that the tumoural cells are effectively destroyed or killed, while sparing as much as possible the healthy cells, in particular those adjacent to the tumoural cells. A first step of a treatment plan is the capture of images of the tumoural region by CT-scan. Based on these images, an oncologist identifies the right targets and determines the locations and doses to be deposited to kill the tumoural cells. Such plan must satisfy multiple, often competing, parameters, and is therefore quite complex. For this reason, treatment planning is generally carried out with a computer.

The treatment plan (TP) generally comprises a definition of an array of n beamlets (bi), including values of planned parameters comprising,
- a planned position of each beamlet, defining the positions of spots aimed at by each beamlet which depends *inter alia* on the positions, sizes, and geometries of the tumoral cells within the structure of interest,
- a planned monitor unit (MUpi) of each beamlet, which relates to the number of particles going through the nozzle of the particle accelerating system and which must reach a given spot in the structure of interest.
- a planned beamlet scanning sequence over the planned positions (Xpi); this is important, since a beamlet deposits a dose into the corresponding spot, but can also deposit lower doses into neighbouring spots, which cannot be neglected when accounting the overall doses deposited in each spot; this is particularly true for flash treatment described more in detail *infra.*

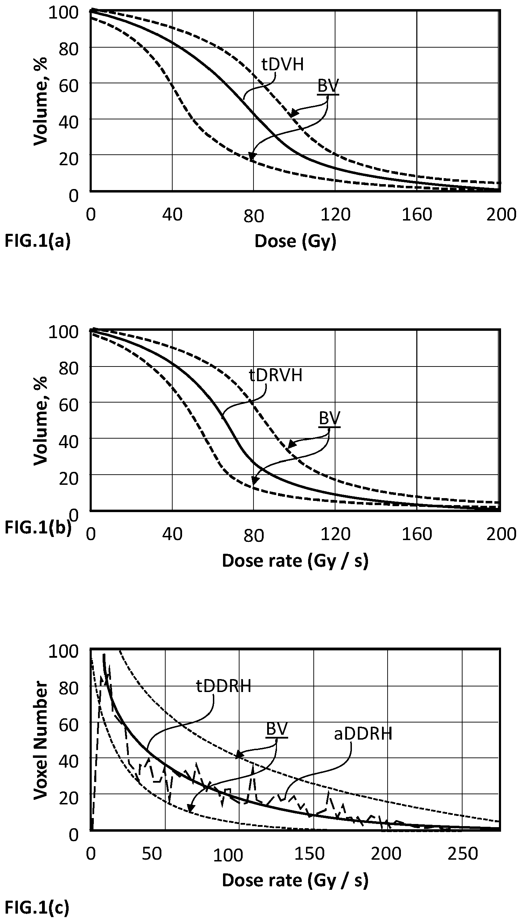

The treatment plan must ensure that, at the end of the treatment, a total target dose greater than or equal to a minimum target dose has been delivered to the tumoural cells forming the target effective for destroying / killing the tumoural cells. This can be defined by a target dose volume histogram (= tDVH), for the structure of interest. An example of tDVH is represented with a solid line in Figure 1(a), showing a graph plotting the volume (%) of the structure of interest which must receive at least a target dose as defined by the abscissa of the curve of Figures 1(a). The oncologist also defines an acceptable band of variation (BV) within which the DVH can deviate from the targets tDVH and represented in Figure 1(a) with dashed lines.

Historically, treatment plans by radiation therapy included the delivery of radiation doses to the treated cells at a conventional dose deposition rate (CDR) lower than 1 Gy / s. With rare exceptions, current radiation therapy facilities deliver dose-rates around 0.1 Gy / s and most clinical protocols involve daily delivery of several target fraction doses of 2 to 15 Gy cumulated to reach the total target dose which often exceeds the tolerance limit of normal tissues located in the radiation field, thus damaging them together with the tumoural cells. Recently, it has been observed that a same dose had different effects on healthy cells but not on tumoral cells when deposited at conventional dose deposition rates (CDR) or at ultra-high dose deposition rate (HDR); HDR can be one or more orders of magnitude largerthan conventional dose deposition rates (CDR) usually applied. Deposition of a charge at ultra-high dose deposition rates (HDR) is also referred to as FLASH-radiotherapy (= FLASH-RT). It has been demonstrated experimentally on animals and on various organs, that ultra-high rate dose deposition at HDR can significantly spare healthy tissues in comparison with conventional deposition of a same dose at CDR and, at the same time, tumoural cells respond same or even better to HDR deposition than to CDR deposition. For example, FLASH-RT reportedly elicits in mice a dramatic decrease of the incidence of lung fibrosis, of memory loss subsequent to brain irradiation, and of necrosis of the small intestine whilst keeping the anti-tumour efficiency unchanged. Such specific normal tissue sparing has been confirmed in large animals and a patient with cutaneous lymphoma has already been treated with FLASH-RT.

The dose rate distribution in tissue can be defined by a target dose rate volume histogram (= tDRVH), for the structure of interest. An example of tDRVH is represented with a solid line in Figure 1 (b), showing a graph plotting the volume (%) of the structure of interest which must receive a target dose rate or higher for the tDRVH as defined by the abscissa of the curves of Figure 1 (b). The oncologist also defines an acceptable band of variation (BV) within which the DRVH can deviate from the target tDRVH and represented in Figure 1(b) with dashed lines.

The DVH and DRVH are cumulative histograms. There are other ways, however, of representing the distribution of doses or dose rates. For example, Figure 1(c) shows a differential dose rate histogram (DDRH) indicating the number of voxels in a structure of interest which receive a dose at the corresponding dose rate indicated in the abscissa. The acceptable band of variation (BV) is represented with dashed lines, and the long-dashed line is actual values (= aDDRH) measured during a treatment session. Other representations are possible. All types of representations of the desired doses and dose rates distributions in a structure of interest a treatment plan must achieve are herein collectively referred to as "dose distribution histograms (DDH)" and "dose rate distribution histograms (DRDH)." The expression "dose (rate) distribution histogram (D(R)DH)" is also used herein for sake of conciseness to include both DDH and DRDH.

Fulfilling the planned beamlet scanning sequence may require defining a planned starting time and a planned end time for each beamlet. This is particularly important for FLASH-RT.

A treatment plan system (TPS) defines beamlets parameters including the positions (Xj), monitoring units (MUj) and spots sequence to achieve the treatment plan (TP). A translation system (= TS) determines the operating parameters of a given particle accelerating system required for implementing the beamlets parameters, taking into account limits of the particle accelerating system. This is described, e.g., in US20200298020, EP3932482, and EP3932481A1. The operating parameters are determined to ensure that the beamlets delivered by the given particle accelerating system will deposit doses into the structure of interest according to the target dose (rate) distribution histogram (D(R)DH) within the acceptable bands of variation (BV). The conversion of the TP into machine operating parameters is very important to ensure that a treatment session can be completed within the treatment plan, and not interrupted because at some point, the random variability of some operating parameters leads the beamlets actually delivered by the particle accelerating system to yield a D(R)DH which falls out of the acceptable band of variation (BV).

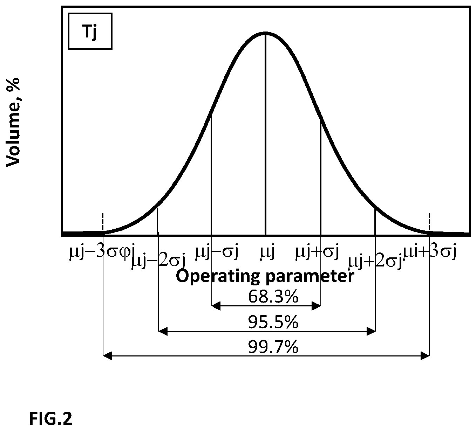

The nominal value of the operating parameters defined in the plan may not be precisely met by a given particle accelerating system. The operating parameters values at which the particle accelerating system will actually be operating follow instead specific statistical distributions (Tj) as illustrated in Figure 2, characterized by an average value (µj) (which is the nominal value defined by the treatment plan) and a variance (*σj*²) representing the random variability of the treatment machine. This means that, even if the TS correctly converts the treatment plan (TP) into operating parameters to ensure that the given particle accelerating system delivers beamlets characterized by the average values (µj), the actual values of the operating parameters on a specific treatment session will be spread around the average value (µj) over the statistical distribution curve (Tj) (cf. Figure 2). It is therefore clear that during a treatment session, the operating parameters will deviate from the average values (µi) following the distributions thereof. In some cases, values of the actual operating parameters of the beamlets may yield D(R)DH extending beyond (outside) the corresponding acceptable bands of variation (BV), and the treatment session must be interrupted despite a correct TP conversion by the TS. If a treatment session does not proceed as planned, it can be dangerous for the patient.

Some particle accelerating systems are equipped with a monitoring device measuring the actual operating parameters of the beamlets as they are being delivered through a nozzle. EP2116277, EP3375484, US10456598, EP3222322, WO2020249565, and EP2833970 describe examples of devices for *in situ* monitoring and verification of a selection of operating parameters of the beamlets delivered by a particle accelerating system. In case the operating parameters of one or more beamlets thus monitored differ from the planned values (which is likely to happen), there is a risk that the corresponding D(R)DH extend beyond the acceptable band of variation (BV). Should this be the case, the treatment session must be stopped. This is very uncomfortable for the patient who may have to come back later to complete the treatment session, depending on the usually tight schedule of the particle accelerating system. It is therefore important to take the distributions of the operating parameters into consideration to ensure that a treatment session can be completed within a predefined confidence level (CLj) with all beamlets yielding the D(R)DH in accordance with the treatment plan.

Furthermore, an actual value of an operating parameter differing from the corresponding planned value does not necessarily mean that the corresponding D(R)DH extend beyond the acceptable band of variation. With the calculating power of processors available to date, it is not thinkable of calculating for each measurement of the actual values of the operating parameters the corresponding calculated D(R)DH to decide whether or not to interrupt the treatment session because one actual value differs from the planned values of the operating parameters.

EP3498336 describes a system and method for treating a dummy (mannequin) and evaluating the DVH by dosimetry before applying the treatment to a patient. This technique clearly reduces the risk of having to interrupt a treatment session, but it also requires blocking a particle accelerating system for the time required to make the tests, during which it cannot be used for treating a patient. Furthermore, a dosimetric tolerance can translate into different machine tolerance levels for spots at different locations or with different MUs (e.g., spot on the edge of the structure of interest may have a higher constraint on position accuracy than spots at the centre of the structure). It is also not obvious how to translate a dose rate tolerance into a tolerance for each spot in the spot map. In some places it is more important to check that the irradiation is in FLASH-RT mode than in other places. The tolerances on the dose rate may also be different between positions in the tissue. For example, FLASH-RT could be required at the edges of the structure of interest, where tumoral cells are flanked by healthy cells which must be spared.

To date, most methods for ensuring that the operating parameters will yield the desired D(R)DH is a *posteriori,* i.e., by measuring the treatment properties of the beamlets delivered by the particle accelerating system or, at best, based on dosimetric tests on a dummy (mannequin) performed before treating the patient. There remains a need in the art for a method for determining by calculation, i.e., without having to use precious accelerator time and energy, a set of operating parameters of the particle accelerating system to be used, yielding within a predefined confidence level the desired D(R)DH within the acceptable band of variation (BV).

The present invention proposes a computer implemented method for optimizing tolerance values of operating parameters of a particle accelerating system allowing a beam formed by a plurality of beamlets of accelerated particles to deposit doses by pencil beam scanning (= PBS) to a patient according to a treatment plan (= TP). The method allows determining the confidence level (CLj) for a given particle accelerating system to deliver beamlets which will fulfil the TP as a function of a set of operating parameters. If the confidence level thus obtained is too low, an alternative set of operating parameters needs be evaluated. These and other advantages of the present invention are presented in continuation.

### SUMMARY OF THE INVENTION

The present invention is defined in the appended independent claims. Preferred embodiments are defined in the dependent claims. In particular, the present invention concerns a method implemented by a computer for optimizing tolerance values of operating parameters of a particle accelerating system allowing a beam formed by a plurality of beamlets of particles accelerated along an irradiation axis (Z), to deposit doses by pencil beam scanning (= PBS) into a structure of interest of a patient according to a treatment plan (= TP), the computer implemented method comprising,
(a) providing an input comprising
   ∘ the treatment plan (= TP) including a definition of an array of beamlets (bi), characterized by planned parameters, including,
      ▪ a planned position (Xpi) of each beamlet (bi) on a plane (X,Y) normal to the irradiation axis (Z),
      ▪ a planned monitor unit (MUpi) of each beamlet,
      ▪ a planned beamlet scanning sequence over the planned positions (Xpi),
   ∘ a planned starting time (t0pi) and end time (t1 pi) at which each beamlet is to be delivered,
   o a definition of the structure of interest, defining one or more tissues being traversed by a number of the beamlets (bi),
   ∘ values of one or more target dose (rate) distribution histograms (= tD(R)DH) including a dose distribution histogram (= tDDH) and / or a target dose rate distribution histogram (= tDRDH), for the structure of interest obtained with a treatment with the planned parameters, wherein the dose distribution histogram (DDH) preferably is a dose distribution volume histogram (DDVH), and the dose rate distribution histogram (DRDH) preferably is a dose rate distribution volume histogram (DRDVH) or a differential dose rate histogram (DDRH).
   ∘ acceptable bands of variation (BV) in which the one or more tD(R)DH are allowed to vary,
(b) providing a tentative statistical distribution (Tj) of operating parameters of the particle accelerating system centred on corresponding average values (µj) representative of the performance of the particle accelerating system and defining a confidence level (CLj) of the tentative statistical distribution (Tj), wherein the operating parameters comprise,
   ∘ the monitor unit (MUj) of each beamlet,
   ∘ the position of each beamlet (Xj), and
   ∘ the starting time (t0j) and end time (t1j) of delivery of each beamlet,
(c) randomly selecting from the corresponding tentative statistical distributions (Tj) within the predefined confidence levels (CLj) a value (MUij) of the monitor unit, a value (Xij) of the position of the beamlet, and a value (t0ij, t1 ij) of each of the starting time (t0ij) and end time (t1 ij),
(d) calculating one or more calculated dose (rate) distribution histograms (= cD(R)DHj-Run x) with the values thus randomly selected,
(e) repeating the last two steps (c), (d) a statistically representative number of times (N) to yield calculated distributions (CDj) characterizing the one or more thus calculated cD(R)DHj-Run x for all the values thus randomly selected of the operating parameters,
(f) comparing the calculated distributions (CDj) of the one or more cD(R)DHj with the corresponding acceptable bands of variation (BV) and determining whether the calculated distributions (CDj) of the one or more cD(R)DHj are comprised in the corresponding acceptable bands of variation within the pre-defined confidence level (CLj).

A final statistical distribution (Tf) can be set with a final confidence level (CLf) to the corresponding operating parameters by a human operator or by a processor as follows,
- If the calculated distributions (CDj) of the one or more cD(R)DHj are all comprised within the corresponding acceptable bands of variation (BV) with the pre-defined confidence level (CLj), for the given treatment plan (TP), setting the tentative statistical distribution (Tj) as the final statistical distribution (i.e., Tf = Tj) and the confidence level (CLj) as the corresponding final confidence level (CLf = CLj) to define the corresponding operating parameters
- If any one of the one or more cD(R)DHj calculated with one set of randomly selected values within the corresponding confidence levels (CLj) of the statistical distribution (Tj) of the operating parameters extends beyond the corresponding acceptable bands of variation (BV), repeating steps (b) to (f) as defined supra,
   ∘ with new tentative statistical distributions (T(j+k)) of the operating parameters and / or
   ∘ selecting new, less ambitious confidence levels (CL(j+k)),
until the calculated distributions (CD(j+k)) of the one or more cD(R)DH(j+k) and are all comprised within the corresponding acceptable bands of variation (BV), and setting the tentative statistical distribution (T(j+k)) as the final statistical distribution (i.e., Tf = T(j+k)) and setting the corresponding confidence level (CL(j+k) as the final confidence level (CLf = CL(j+k)) to define the corresponding operating parameters.

The new tentative statistical distributions (T(j+k)) can have lower standard deviations (*σj*) than the corresponding tentative statistical distributions (Tj) defined supra. The tentative statistical distributions (Tj) of each of the operating parameters are preferably gaussian distributions, and the value of the confidence level (CLj) can be comprised between 68% and 99.7%, preferably between 95.5 and 99% of the tentative statistical distribution. Note that a confidence level (CLj) of 68% corresponds to µj ± *σj,* a confidence level (CLj) of 95% corresponds to µj ± 2*σj*, and a confidence level of 99.7% of the tentative statistical distribution, corresponds to µj ± 3*σj*, wherein µj is an average value and *σj* is the standard deviation of the corresponding tentative statistical distributions. The average values (µj) and standard deviations (*σj*) of each operational parameter can be different for each beamlet (bi).

The particle accelerating system can be equipped with a cyclic check module configured for measuring at different intervals or continuously actual values of the operating parameters including the monitor unit (MUai), the position (Xai), and of the starting time and end time (t0ai, t1ai) of the beamlets emitted by the particle accelerating system. The particle accelerating system can also be equipped with a processor configured for comparing the actual values of the operating parameters with the corresponding confidence level (CLj), and for stopping a treatment session in case one actual value of an operating parameter falls out of the corresponding confidence level (CLj).

The planned parameters also comprise a planned beamlet size (dj) and a planned beam current (Ij), whose respective values (dij, lij) used for calculating the one or more calculated dose (rate) distribution histograms (= cD(R)DH) are randomly selected within a corresponding tentative statistical distribution (Tj) of the beamlet size (dj) and of the planned beam current (Ij).

The calculated distributions (CDj) of the one or more cD(R)DH are defined by a corresponding area comprised in an envelope defined between, on the one hand, a minimum calculated dose (rate) distribution histogram (= cD(R)DHj0) and, on the other hand, a maximum calculated dose (rate) distribution histogram (= cD(R)DHj1). The cD(R)DHj0 is defined by the lowest values of cD(R)DHj calculated with the predefined confidence level (CLj) from the N randomly selected values of the monitor unit (Muij), position (Xij) of the beamlets, and starting time and end time (t0ij, t1 ij) and the cD(R)DHj1 is defined by the highest values of cD(R)DHj calculated with the predefined confidence level (CLj) from the N randomly selected values of the monitor unit (Muij), position (X0i) of the beamlets, and starting time and end time (t0ij, t1 ij).

The treatment plan can include depositing doses into at least a portion of the structure of interest at ultra-high deposition rates (UHDR) defined as a deposition rate greater than or equal to 1 Gy / s.

The present invention also concerns an error predicting module configured for implementing the method as defined supra, comprising,
- a memory comprising a plurality of tentative statistical distributions (Tj) for each operating parameter centred on a plurality of corresponding average values (µj),
- a user interface configured for,
   ∘ entering the treatment plan (TP) including one or more target dose (rate) distribution histograms (= tD(R)DH) including the target dose distribution histogram (= tDDH) and / or the target dose rate distribution histogram (= tDRDH), as well as the corresponding acceptable bands of variation (BV),
   ∘ selecting from the memory or entering a planned starting time (t0pi) and end time (t1 pi) at which each beamlet is to be delivered
   ∘ selecting from the memory or entering for each beamlet, a first tentative statistical distribution (Tj) for each operating parameter, including the monitor unit (MUj), the position (Xj) of the beamlet, and starting time and end time (t0i, t1i),
   ∘ entering a confidence level (CLj) on the operational parameters,
- a processor configured for,
   (i) randomly selecting a value of the monitor unit (MUij), a value of the position (Xij) of the beamlet, values of the starting time and end time (t0ij, t1 ij) comprised within the predefined confidence levels (CLj) of the corresponding tentative statistical distributions (Tj),
   (ii) calculating one or more of a calculated dose distribution histogram (= cDDHj) and a calculated dose rate distribution histogram (= cDRDHj) with the values thus randomly selected,
   (iii) repeating the last two steps a statistically representative number of times (N) to yield the calculated distributions (CDj) of the thus calculated cDDHj and cDRDHj for each beamlet.

The processor can be further configured, in case any one of the one or more of the calculated distributions (CDj) of cDDHj and cDRDHj are not comprised within the corresponding acceptable bands of variation with the pre-defined confidence level (CLj), for repeating steps (i) to (iii) supra with new tentative statistical distributions (T(j+k)) of the operating parameters, until the calculated distributions (CDj) of the one or more of cDDHj and cDRDHj are both comprised within the corresponding acceptable bands of variation with the pre-defined confidence level (CLj).

### BRIEF DESCRIPTION OF THE FIGURES

For a fuller understanding of the nature of the present invention, reference is made to the following detailed description taken in conjunction with the accompanying drawings in which:
**Figures 1(a) to 1(c)****:** show examples of tDVH, tDRVH, and tDDRH curves with their corresponding acceptable bands of variation (BV).
**Figure 2****:** shows a Gaussian distribution of an operating parameter of a given particle accelerating system.
**Figures 3(a) to 3(c)****:** show various steps of the method of the present invention, with a positive result.
**Figures 4(a) to 4(c)****:** show various steps of the method of the present invention, with a negative result.
**Figures 5(a) to 5(d)****:** show N runs of calculations yielding the calculated distributions (CDj) of the thus calculated cD(R)DHj as shown in Figures 3(b)&3(c).
**Figure 6****:** shows a flowchart with the various steps of the method of the present invention.

### DETAILED DESCRIPTION OF THE INVENTION

The present invention concerns a computer implemented method and an error predicting module which considerably reduce the risk of carrying out a treatment session which does not respect a corresponding treatment plan, for reasons of equipment.

### Method for optimizing tolerance values of operating parameters

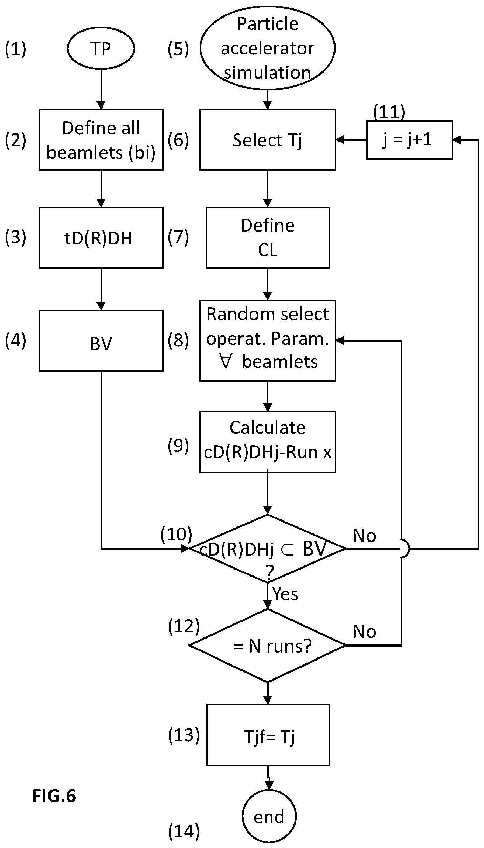

The present invention concerns a method implemented by a computer for optimizing tolerance values of operating parameters of a particle accelerating system allowing a beam formed by a plurality of beamlets of particles accelerated along an irradiation axis (Z), to deposit doses by pencil beam scanning (= PBS) to a patient according to a treatment plan (= TP). The particles are preferably protons, but they can be electrons, heavy ions beams, but also waves formed by accelerated particles interacting with a converting material, such as x-rays (or *γ*-rays). As illustrated in Figure 6, the computer implemented method requires the input of a number of values of planned parameters. These can be provided by a treatment plan (TP) including the following planned parameters,
- a planned position (Xpi) of each beamlet (bi) on a plane (X,Y) normal to the irradiation axis (Z),
- a planned monitor unit (MUpi) of each beamlet,
- a planned beamlet scanning sequence over the planned positions (Xpi),

The method also requires a planned starting time (t0pi) and end time (t1 pi) at which each beamlet is to be delivered. This is particularly important in case of FLASH-RT. The starting and end times may or may not be part of the TP.

The structure of interest must be defined, characterizing one or more tissues being traversed by or interacting with the beamlets (bi). The structure of interest comprises the target comprising the tumoural cells to be killed, but also healthy tissues traversed or somehow touched by one or more beamlets. These include for example, the tissues located upstream from the target along the irradiation axis (Z), i.e., between the nozzle of the particle accelerating system and the target, or also the tissues adjacent to and surrounding the target.

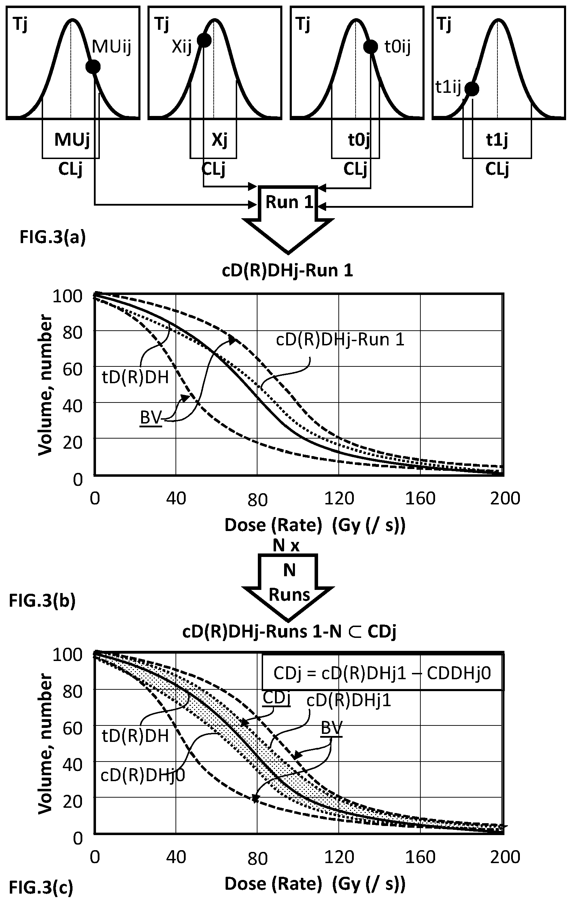

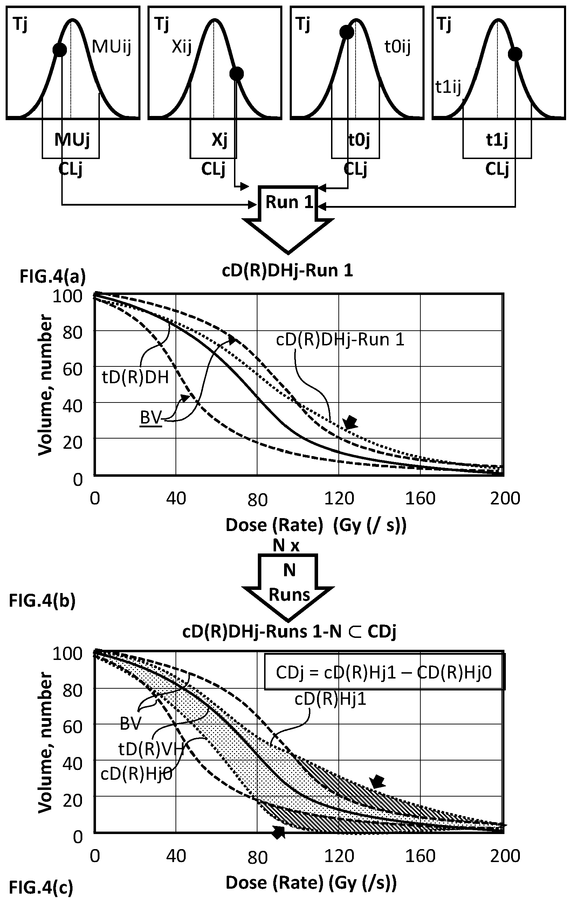

An objective of the treatment is to yield at the end of a session, given values of a target dose (rate) distribution histogram (tD(R)DH) for the structure of interest, within an acceptable band of variation (BV) in which tD(R)VH are allowed to vary. As shown in Figures 1(a) to 1(c), tD(R)DH can include for example a target dose volume histogram (= tDVH) (cf. Figure 1(a)), a target dose rate volume histogram (= tDRVH) (cf. Figure 1(b)), or a target differential dose rate histogram (tDDRH) (cf. Figure 1(c)). Note that other histogram representations are possible and included in the term tD(R)DH, to display the information on the dose and dose rate distributions. Figures 1(a) to 1(c) show examples of tDVH, tDRVH, and tDRDH represented by the solid lines, as well as of the corresponding acceptable bands of variation (BV) represented by the area comprised between the dashed lines. Similar acceptable bands of variation can be defined for any type of alternative D(R)DH histogram representation. If the beamlets delivered by the particle accelerating system achieve DVH and DRVH comprised within the acceptable band of variation as shown in Figures 3(b) and 3(c), the session is successful. In case the DVH or DRVH fall out of the acceptable band of variation (BV) as shown in Figures 4(b) and 4(c), the treatment session must be interrupted, to the great discomfort of the patient who must wait for a free slot in the particle accelerator system's schedule to resume the treatment session.

Figure 1(c) includes the actual values of the dose rate distribution histogram (aDDRH) obtained upon measuring the beamlets parameters during a treatment session (cf. long-dashed line). It can be seen that the curve aDDRH in Figure 1(c) is fully enclosed within the acceptable band of variation (BV). The treatment session was therefore successful. Had the curve aDDRH extended beyond the acceptable band of variation (BV), however, the treatment session would have had to be interrupted. In order to decrease the risk of having to interrupt a treatment session because of the actual D(R)DH falling out of the acceptable band of variation, the present invention proposes to implement the following actions with a computer.

First, as shown in Figures 3(a) and 4(a), a tentative statistical distribution (Tj) is selected of operating parameters (MUj, Xj, t0j, t1j) of the particle accelerating system centred on corresponding average values (µj) representative of the performance of the particle accelerating system. A confidence level (CLj) of the tentative statistical distribution (Tj) of each of the operating parameters is defined. The operating parameters include:
o the monitor unit (MUj) of each beamlet,
o the position of each beamlet (Xj), and
o the starting time (t0j) and end time (t1j) of delivery of each beamlet.

The selection of tentative statistical distributions (Tj) is based on the performance of the particle accelerating system and is expected to generate a beam delivering the beamlets scanning sequence and generating calculated dose volume histograms (= cDVHj) and calculated dose rate volume histograms (= cDRVHj in the structure of interest that are comprised within the acceptable band of variation (BV). For example, a treatment plan system (TPS) can determine average values (µi) achievable by the particle accelerating system which would yield the desired cDVH and cDRVH. The actual operating parameters of the treatment machine on a specific day, however, are not restricted to the corresponding average values (µi) but are distributed generally over a Gaussian curve, varying from day to day or during the course of a day (cf. Figure 2). The resulting cD(R)DH histograms will therefore vary depending on the actual values of the operating parameters at the specific time of the treatment and must therefore be calculated taking account of the distribution. It would not be practical to consider all the possible values of each operating parameter defined by the corresponding distribution curves. A confidence level (CLj) can be defined, restricting the distribution to boundaries which are considered as acceptable. For example, the confidence level can be defined with respect to the standard deviation (*σ*j), such as for example, µj ± n *σ*j, with n = 1 to 3.

A value (MUij) of the monitor unit, a value (Xij) of the position of the beamlet, and values (t0ij, t1 ij) of the starting time (t0ij) and end time (t1 ij) are randomly selected from the corresponding tentative statistical distributions (Tj) within the corresponding confidence levels (CLj); as shown with the black circles in the Gaussian distribution curves (Tj) of Figures 3(a) and 4(a). The values are selected within the confidence level (CLj) previously defined.

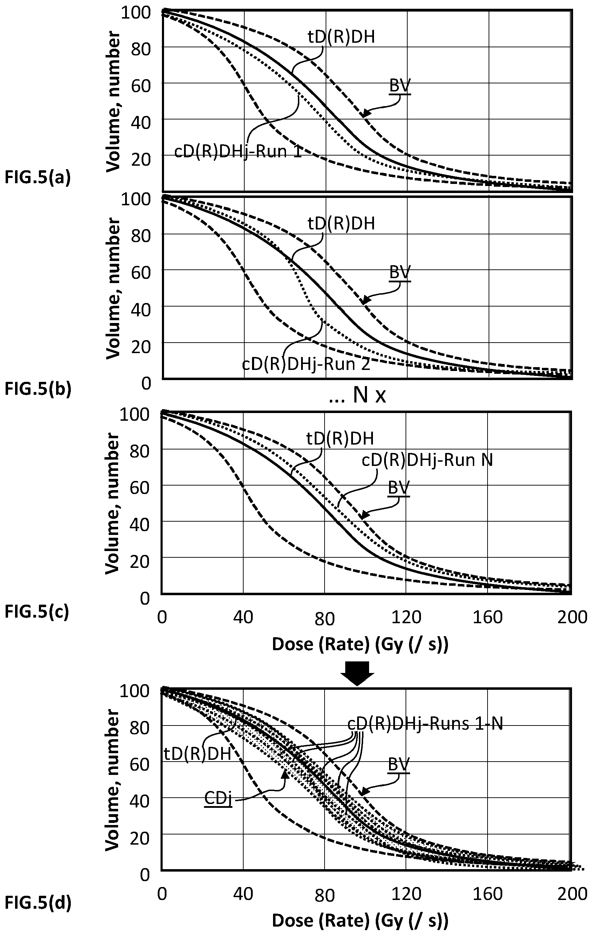

As shown in Figure 5(a), in a first Run (= Run 1), the calculated dose (rate) distribution histogram (= cD(R)DHj) is calculated with the values thus randomly selected. If the calculated cD(R)DHj is enclosed within the corresponding acceptable band of variation, new values of the operating parameters are randomly selected from the tentative statistical distributions (Tj) and the corresponding cD(R)DHj is calculated in a second Run (= Run 2) as illustrated in Figure 5(b). These operations are repeated a statistical number of times (N) as illustrated in Figure 5(c), "Run N", as long as the calculated cD(R)DH-Run x are enclosed within the acceptable band of variation (BV). After N runs, the N curves "cD(R)DH-Run x" with x = 1 to N define in combination a calculated distribution (CDj), represented in Figures 3(b)&3(c), 4(b)&4(c), and 5(d) by the shaded areas bounded by the dotted lines.

If, on the other hand, any one of the cD(R)DHj-Run x thus calculated extends beyond the acceptable band of variation (BV), it could be concluded that there would be a risk higher than the predefined confidence level (CLj) of having to interrupt a treatment run with the operating parameters of the particle accelerating system according to the tentative statistical distributions (Tj). A new tentative statistical distribution (T(j+1)) of the operating parameters are then selected, and the cD(R)DH(j+1) is calculated in a new series of N Runs with randomly selected values of the new tentative statistical distributions (T(j+1)) at each successive Run. This operation is repeated with new tentative statistical distributions (T(j+k)) until the corresponding calculated distributions (CD(j+k)) are entirely comprised within the acceptable band of variation (BV).

Besides the planned position (Xpi), the planned monitor unit (MUpi), the planned beamlet scanning sequence over the planned positions (Xpi), and the planned starting time (t0pi) and end time (t1 pi), the planned parameters can also comprise a planned beamlet size (dj) and a beam current (Ij), whose values (dij, lij)) used for calculating the calculated dose (rate) distribution histogram (= cD(R)DHj) are randomly selected within a corresponding tentative statistical distribution (Tj) of the beamlet size (dj) and beam current (Ij).

The calculated distributions (CDj) of the cD(R)DHj can be defined by a corresponding area comprised between, on the one hand,
- a minimum calculated dose (rate) distribution histogram (= cD(R)DHj0) and, on the other hand,
- a maximum calculated dose (rate) distribution histogram (= cD(R)DHj1),
wherein
- cD(R)DHj0 is defined by the lowest values of cD(R)DHj calculated with the predefined confidence level (CLj) from the N randomly selected values of the monitor unit (Muij), position (Xij) of the beamlets, and starting time and end time (t0ij, t1 ij) and wherein
- cD(R)DHj1 is defined by the highest values of cD(R)DHj calculated with the predefined confidence level (CLj) from the N randomly selected values of the monitor unit (Muij), position (X0i) of the beamlets, and starting time and end time (t0ij, t1 ij).

By comparing the calculated distributions (CDj) of cD(R)DHj with the acceptable band of variation (BV), it can be determined whether or not the calculated distribution (CDj) of cD(R)DHjj is comprised in the corresponding acceptable band of variation for the pre-defined confidence level (CLj). Figures 3(b) and 3(c) show an example of tentative statistical distributions (Tj) with associated confidence level (CLj) which yields calculated distributions (CDj) of cD(R)DHj comprised within the corresponding acceptable bands of variation (BV). By contrast, Figures 4(b) and 4(c) show an example of tentative statistical distribution (Tj) with associated confidence level (CLj) which yield calculated distributions (CDj) of cD(R)DHj extending beyond the corresponding acceptable bands of variation (BV) as indicated by the shaded areas and black arrows. For example, it can be seen in Figure 4(b), that the cD(R)DHj-Run 1 already extends beyond the acceptable band of variation (BV). Calculation with the corresponding tentative statistical distribution can be stopped and a new tentative statistical distribution (T(j+1)) can be selected to repeat the N runs with values of the operating parameters randomly selected from the new tentative statistical distribution (T(j+1)).

The method of the present invention can conclude by setting a final statistical distribution (Tf) to the corresponding operating parameters with a final confidence level (CLf). If the N runs of calculated distributions (CDj) of cD(R)DHj are comprised within the corresponding acceptable band of variation (BV) with the pre-defined confidence level (CLj), for the given treatment plan (TP), the tentative statistical distribution (Tj) can be set as the final statistical distribution (i.e., Tf = Tj) with the final confidence level (CLf = CLj) to define the corresponding operating parameters. It can be concluded that, by implementing the final statistical distributions (Tf) of operating parameters, the particle accelerating system has a probability equal to the final confidence level (CLf) of delivering the beamlets fulfilling the treatment plan (TP) with corresponding actual dose (rate) distribution histogram(aD(R)DH) comprised within the acceptable band of variation (BV), as shown e.g., in Figure 1(c).

If, on the other hand, any one of the N runs of cD(R)DHj extends beyond the boundaries of the corresponding acceptable bands of variation (BV) with the pre-defined confidence level (CLj) as shown e.g., in Figure 4(b), a new tentative statistical distribution (T(j+1)) and / or a new confidence level (CL(j+1)) can be defined for one or more of the operating parameters and the cD(R)DHj can be calculated N times with N randomly selected values of each of the operating parameters. These operations can be repeated as often as necessary with new tentative statistical distributions (T(j+k)) of the operating parameters and / or with alternative confidence levels (CL(j+k)), until the calculated distributions (CD(j+k)) of cD(R)DHj(j+k) is comprised within the corresponding acceptable band of variation (BV) for the pre-defined confidence level (CLj) or (CL(j+k)). The final statistical distribution (Tf) can thus be set as being the tentative statistical distribution (T(j+k)) (i.e., Tf = T(j+k)) and the corresponding confidence level (CL(j+k)) can be set as the final confidence level (CLf = CL(j+k)), to define the corresponding operating parameters.

The new tentative statistical distributions (T(j+k)) defined in case the j+(k-1) preceding tentative statistical distributions did not yield calculated distributions included within the bands of variation (BV) surrounding tD(R)DH for the pre-defined confidence level (CLj), can be selected as distributions having lower standard deviations (*σj*) (or variances (*σj*²)) than the corresponding preceding tentative statistical distributions (Tj+(k-1)).

Setting the final statistical distribution (Tf) for the corresponding operating parameters can be carried out by a human operator or automatically, by a processor.

The tentative statistical distributions (Tj) of each of the operating parameters are preferably gaussian distributions. The values of the confidence levels (CL) are preferably comprised between 68% and 99.7%, preferably between 95.5 and 99% of the tentative statistical distribution. As shown in Figure 2, a confidence level (CLj) of 68% corresponds to µj ± *σj.* A confidence level (CLj) of 95% corresponds to µj ± 2*σj* and a confidence level of 99.7% of the tentative statistical distribution, corresponds to µj ± 3*σj*, wherein *σj* is the standard deviation of the corresponding tentative statistical distributions. For example, in terms of absolute deviations, the position (Xj) of a spot may, for example, vary by ±1 mm from the average value µj of the Xj. The monitor unit (MUj) can for example vary by about 0.5% around the average value (µj) of MUj. Note that the average values (µj) and standard deviations (*σj*) of each operational parameter can be different for each beamlet (bi).

The particle accelerating system can be equipped with a cyclic check module configured for measuring at different intervals or continuously actual values of the monitor unit (MUai), the position (Xai), and of the starting time and end time (t0ai, t1ai) of the beamlets emitted by the particle accelerating system. A processor can be configured for determining whether any actual operating parameter falls out of the corresponding final confidence level (CLf) (cf. Figures 3(a) and 4(a), showing the confidence levels (CLj) spanning over a portion only of the statistical distributions (Tj)). In such case, an alarm can be triggered informing an operator of this event. As a safety measure, the processor can also be configured for stopping the treatment as soon as an operating parameter falling out of the confidence level (CLj) is detected.

The fact that one or more values of the actual operating parameters fall out of the corresponding confidence levels (CLj) does not mean that the resulting aD(R)DH necessarily falls out of the acceptable band of variation (BV). It could therefore be over-shooting to interrupt the treatment session simply because one actual value of any operating parameter fell out of the corresponding confidence level (CLj), as it could perfectly yield a D(R)DH comprised within the acceptable band of variation (BV). The processor may be configured for calculating the cD(R)DH as soon as a measured actual value of an operating parameter falls out of the corresponding confidence level (CLj) to determine whether or not they are comprised within the corresponding acceptable bands of variation (BV). The cD(R)DH can be calculated based on the actual values of the operating parameters measured on the beamlets already delivered, including the parameter falling out of the confidence level (CLj), and on the average values (µj) of the operating parameters according to the final statistical distribution (Tf) for the beamlets which remain to be delivered to end the treatment session. If the thus calculated cD(R)DH falls out of the acceptable band of variation (BV), the treatment session is stopped. If, on the other hand, the calculated cD(R)VH is within the acceptable band of variation (BV), the treatment session can proceed further. With this functionality of the processor, the risk of stopping a treatment session is further reduced.

This function does not take excessive calculating power, as it would concern only 100% - CLj % of the actual values of the operating parameters. For a confidence level (CLj) of 95% i.e., µj ± 2*σj,* there would be a probability of only 5% that a value of an operating parameter should fall out of the confidence level (CLj) whch the D(R)DH would have to be calculated for. For a confidence level of 99.7% of the tentative statistical distribution, i.e., µj ± 3*σj*, it would concern a probability of merely 0.3% where such calculation would be required.

In a preferred embodiment, the treatment plan includes FLASH-RT, in that doses are to be deposited into at least a portion of the structure of interest at ultra-high deposition rates (UHDR) defined as a deposition rate greater than or equal to 1 Gy / s. In this embodiment, the planned parameters also comprise a beam current (I), whose values (Ij) used for calculating the calculated dose distribution histogram (= cDDHj) and, in particular, the calculated dose rate distribution histogram (= cDRDHj) are randomly selected within a corresponding tentative statistical distribution (Tj) of the beam current (Ij).

### Error predicting module

The present invention also concerns an error predicting module configured for implementing the method described supra. The error predicting module comprises a memory comprising a plurality of tentative statistical distributions (Tj) for each operating parameter centred on a plurality of corresponding average values (µj). It also comprises a user interface configured for entering,
- planned operators including the treatment plan (TP) including the planned position (Xpi), the planned monitor unit (MUpi), and the planned beamlet scanning sequence, the planned starting time (t0pi) and end time (t1 pi),
- the target dose (rate) distribution histogram (= tD(R)DH) and corresponding acceptable band of variation (BV),
- for each beamlet, a first tentative statistical distribution (Tj) selected among or defined by entering values of the average values (µj) and standard deviations (*σ*j) from one or more of the plurality of tentative statistical distributions for each operating parameter, including at least the monitor unit (MUj), the position (Xj) of the beamlet, and starting time and end time (t0i, t1i),

The error predicting module comprises a processor configured for,
- randomly selecting a value of the monitor unit (MUij), a value of the position (Xij) of the beamlet, values of the starting time and end time (t0ij, t1 ij) comprised within the predefined confidence levels (CLj) of the corresponding tentative statistical distributions (Tj),
- calculating a calculated dose (rate) distribution histogram (= cD(R)Dj) with the values thus randomly selected,
- repeating the last two steps a statistically representative number of times (N) to yield the calculated distributions (CDj) of the thus calculated cD(R)Dj for each beamlet.

The processor can be further configured, in case the calculated distribution (CDj) of cD(R)Dj is not comprised within the corresponding acceptable band of variation with the pre-defined confidence level (CLj), for repeating the foregoing three steps with new tentative statistical distributions (T(j+k)) of the operating parameters, until the calculated distributions (CDj) of cD(R)Dj is comprised within the corresponding acceptable bands of variation with the pre-defined confidence level (CLj).

### Flowchart (Figure 6)

Figure 6 shows a flowchart illustrating the various steps of the method of the present invention. The requirements of the treatment plan (TP) (cf. Figure 6(1)) are translated into planned parameters for all the beamlets (cf. Figure 6(2)). One or more theoretical dose (rate) distribution histograms (tD(R)DH) are defined (cf. Figure 6(3)) with their corresponding acceptable bands of variation (BV) (cf. Figure 6(4)). As defined supra, the dose (rate) distribution histograms (D(R)DH) can include non-exhaustively, the dose volume histogram (DVDH), the dose rate volume histogram (DRVH), or the differential dose rate distribution histogram (DDRH), and the like.

The particle accelerating system is simulated (cf. Figure 6(5)), by defining a first tentative statistical distribution (Tj) and a corresponding confidence level (CLj) (cf. Figure 6, (6)&(7)). A value of each operational parameter is randomly selected within the confidence level (CLj) of the tentative statistical distributions (Tj) (cf. Figure 6(8)) and the resulting cD(R)DHj-Run (x = 1) is calculated (cf. Figure 6(9)). If the calculated CD(R)DH-Run (x = 1) is not within the acceptable band of variation (BV) a new tentative statistical distribution (T(j+1)) is selected with a corresponding confidence level (CL(j+1)) (cf. Figure 6, steps (10) & (11)), and steps (7) to (10) are repeated. If, on the other hand, the calculated CD(R)DH-Run (x = 1) is within the acceptable band of variation (BV), a new value of each operational parameter is randomly selected a statistically representative number of times (N) within the confidence level (CLj) of the tentative statistical distributions (Tj) (cf. Figure 6(11), (12)&(8)) and steps (8) to (10) are repeated. When all N Runs x yielded calculated CD(R)DH-Run (x = 1 to N) are within the acceptable band of variation (BV), the corresponding tentative statistical distribution (Tj) can be set as the final statistical distribution (Tf) (cf. Figure 6(13)). The particle accelerating system can be programmed with the operational parameters according to the corresponding final statistical distributions (Tf). With such programming, the particle accelerating system has a probability of CLj% of delivering beamlets satisfying the treatment plan without interruption of the treatment session.

The method of the present invention can be implemented with a single calculated dose (rate) histogram (cD(R)DH) or with several histograms which must all be within the corresponding acceptable bands of variation (BV) to set the final statistical distribution (Tf). If a first tentative statistical distribution (Tj) yields one histogram (e.g., cDVH) enclosed within the corresponding acceptable band of variation (BV) but another histogram (e.g., cDRVH) extending beyond the acceptable band of variation (BV), a new tentative statistical distribution (T(j+1)) must be selected and the method carried out again, until a tentative statistical distribution (T(j+k)) is found that fits all the required histograms into the corresponding acceptable bands of variation (BV). Dose rate related histograms (e.g., DRVH, DDRH) are particularly important for treatment plans comprising beamlets to be emitted in FLASH-mode for depositing doses at ultra-high deposition rates into selected spots of the structure of interest.

| **Ref** | **Description** |
|---|---|
| BV | Band of variation |
| CDj | Calculated distribution of cDVHj and cDVRHj |
| D(R)DH | Dose (rate) distribution histogram (D(R)DH) includes both "dose distribution histograms (DDH)" and "dose rate distribution histograms (DRDH).". |
| cD(R)Dj | Calculated dose (rate) distribution histogram of beamlet Si with tentative statistical distribution Tj |
| cDVH | Calculated dose volume histogram of beamlet Si with tentative statistical distribution Tj |
| cDRVH | Calculated dose rate volume histogram of beamlet Si with tentative statistical distribution Tj |
| cD(R)Dj0 | Minimum calculated dose (rate) distribution histogram |
| cD(R)Dj1 | Maximum calculated dose (rate) distribution histogram |
| CLj | Confidence level |
| CLf | Final confidence level |
| Dj | Beamlet size |
| DRj | Dose deposition rate |
| i | i = 1 - N Runs |
| j, k | Identifies one tentative statistical distribution of an operational parameter |
| MUai | Actual monitor unit of a beamlet Si as delivered by the particle accelerator |
| MUj | Monitor unit distribution of a beamlet Si according to the tentative statistical distribution |
| MUij | Random value from the monitor unit distribution of a beamlet Si |
| MUpi | Planned monitor unit of a beamlet Si |
| PBS | Pencil Beam Scanning |
| Si | Beamlet |
| Tj, T(j+k) | Tentative statistical distribution of an operational parameter |
| Tf | Final statistical distribution of an operational parameter |
| TP | Treatment plan |
| t0aj | Actual starting time of a bemlet Si |
| t0j | Starting time distribution of a beamlet Si according to the tentative statistical distribution |
| t0ij | Random value from the starting time distribution of a beamlet Si |
| t0pi | Planned starting time of a beamlet Si |
| t1ai | Actual end time of a bemlet Si |
| t1i | End time distribution of a beamlet Si according to the tentative statistical distribution |
| t1ij | Random value from the end time distribution of a beamlet Si |
| t1pi | Planned end time of a beamlet Si |
| tD(R)Dj | Theoretical dose (rate) distribution histogram of beamlet Si with tentative statistical distribution Tj |
| tDVH | Theoretical dose volume histogram of beamlet Si with tentative statistical distribution Tj |
| tDRVH | Theoretical dose rate volume histogram of beamlet Si with tentative statistical distribution Tj |
| Xai | Actual position of a beamlet Si |
| Xi | Position of a beamlet Si |
| Xij | Random value from the position of a beamlet Si |
| Xpi | Planned position of a beamlet Si |
| (X,Y) | Plane normal to the irradiation axis (Z) |
| Z | Irradiation axis |
| | |
| Δti | Duration of dose deposition by beamlet Si |
| µj | Average value of an operational parameter distribution of beamlet Si |
| σj | Standard deviation of an operational parameter distribution of beamlet Si |

## Claims

1. Method implemented by a computer for optimizing tolerance values of operating parameters of a particle accelerating system allowing a beam formed by a plurality of beamlets of particles accelerated along an irradiation axis (Z), to deposit doses by pencil beam scanning (PBS) into a structure of interest of a patient according to a treatment plan (TP), the computer implemented method comprising,
(a) providing an input comprising
∘ the treatment plan (TP) including a definition of an array of beamlets, bi, each defined by planned parameters, including,
▪ a planned position, Xpi, of each beamlet, bi, on a plane (X,Y) normal to the irradiation axis (Z),
▪ a planned monitor unit, MUpi, of each beamlet,
▪ a planned beamlet scanning sequence over the planned positions, Xpi,
∘ a planned starting time, t0pi, and end time, t1pi, at which each beamlet is to be delivered,
∘ a definition of the structure of interest, defining one or more tissues being traversed by a number of the beamlets, bi,
∘ values of one or more target dose distribution histograms, tDDH, or dose rate distribution histograms, tDRDH, for the structure of interest obtained with a treatment with the planned parameters, and
∘ acceptable bands of variation, BV, within which the one or more target dose distribution histograms or target dose rate distribution histograms are allowed to vary,
(b) providing a tentative statistical distribution, Tj, of operating parameters of the particle accelerating system centred on corresponding average values, µj, representative of the performance of the particle accelerating system and defining a confidence level, CLj, of the tentative statistical distributuin, Tj, wherein the operating parameters comprise,
∘ the monitor unit, MUj, of each beamlet,
∘ the position of each beamlet, Xj, and
∘ the starting time, t0j, and end time, t1j, of delivery of each beamlet,
(c) randomly selecting from the corresponding tentative statistical distributions, Tj, within the predefined confidence levels, CLj, a value MUij of the monitor unit, a value Xij of the position of the beamlet, and a value t0ij, t1ij of each of the startingtime, t0ij, and end time t1ij,
(d) calculating one or more calculated dose distribution histograms, cDDHj, run x, or dose rate distribution histograms, cDRDHj, run x, with x = 1 to N being a number of calculation runs, with the values thus randomly selected,
(e) repeating the last two steps (c), (d) a statistically representative number N of times to yield calculated distributions, CDj, defining the one or more thus calculated dose distribution histograms, cDDHj, or dose rate distribution histograms, cDRDHj, for all the values thus randomly selected of the operating parameters,
(f) comparing the calculated distributions, CDj, of the one or more calculated dose distribution histograms, cDDHj, or dose rate distribution histograms, cDRDHj, with the corresponding acceptable bands of variation, BV, and determining whether the calculated distributions, CDj, of the one or more calculated dose distribution histograms, cDDHj, or dose rate distribution histograms, cDRDHj, are comprised in the corresponding acceptable bands of variation within the predefined confidence level, CLj.

2. Method according to claim 1, comprising setting a final statistical distribution. Tf, with a final confidence level, CLf, to the corresponding operating parameters as follows,
• If the calculated distributions, CDj, of the one or more calculated dose distribution histograms, cDDHj, or dose rate distribution histograms, cDRDHj, are all comprised within the corresponding acceptable bands of variation, BV, with the predefined confidence level, CLj, for the given treatment plan (TP), setting the tentative statistical distribution, Tj, as the final statistical distribution Tf = Tj, and the confidence level, CLj, as the corresponding final confidence level CLf = CLj, to define the corresponding operating parameter,
• If any one of the one or more calculated dose distribution histograms, cDDHj, or dose rate distribution histograms, cDRDHj, calculated with one set of randomly selected values within the corresponding confidence levels, CLj, of the statistical distribution, Tj, of the operating parameters extends beyond the corresponding acceptable bands of variation, BV,
∘ repeating steps (b) to (f) of claim 1 with new tentative statistical distributions, T(j+k), of the operating parameters, and or
∘ selecting new, less ambitious confidence levels , CL(j+k), until the calculated distributions, CD(j+k), of the one or more calculated dose distribution histograms, cDDH(j+k) or dose rate distribution histograms, cDRDH(j+k), are all comprised within the corresponding acceptable bands of variation, BV, and setting the tentative statistical distribution, T(j+k), as the final statistical distribution Tf = T(j+k), and setting the corresponding confidence level, CL(j+k), as the final confidence level CLf = CL(j+k), to define the corresponding operating parameters.

3. Method according to claim 2, wherein setting the final statistical distribution , Tf, to the corresponding operating parameters is carried out by a human operator or by a processor.

4. Method according to claim 2 or 3, wherein the new tentative statistical distributions T(j+k) defined in claim 2 in case any one of the one or more calculated dose distribution histograms, cDDHj, or dose rate distribution histograms, cDRDHj, extends beyond the corresponding acceptable bands of variation with the predefined confidence level CLj have lower standard deviations σj than the corresponding tentative statistical distributions Tj defined in step (b) of claim 1.

5. Method according to any one of the preceding claims, wherein the tentative statistical distributions Tj of each of the operating parameters are gaussian distributions, and wherein the value of the confidence level CLj is comprised between 68% and 99.7%, preferably between 95.5 and 99% of the tentative statistical distribution, wherein a confidence level of 68% corresponds to µj ± *σj,* a confidence level of 95% corresponds to µj ± 2*σj*, and a confidence level of 99.7% of the tentative statistical distribution, corresponds to µj ± 3*σj*, wherein µj is an average value and *σj* is the standard deviation of the corresponding tentative statistical distributions.

6. Method according to claim 5, wherein the average values µj and standard deviations σj of each operational parameter are allowed to be different for each beamlet bi.

7. Method according to any one of the preceding claims, wherein the particle accelerating system is equipped with a cyclic check module configured for measuring at different intervals or continuously actual values of the operating parameters including an actual monitor unit MUai, an actual Preposition Xai, and an actual value of a starting time t0ai and end time t1ai of the beamlets emitted by the particle accelerating system.

8. Method according to the preceding claim 7, wherein the particle accelerating system is equipped with a processor configured for comparing the actual values of the operating parameters with the corresponding confidence level CLj, and for stopping a treatment session in case one actual value of an operating parameter falls out of the corresponding final confidence level CLf.

9. Method according to any one of the preceding claims, wherein the planned parameters also comprise a planned beamlet size, dj, whose values, dij, used for calculating the one or more calculated dose distribution histograms cDDHj or dose rate distribution histograms cDRDHj are randomly selected within the corresponding tentative statistical distribution Tj of the beamlet size dj.

10. Method according to any one of the preceding claims, wherein the calculated distributions CDj of the one or more calculated dose distribution histograms, cDDHj, or dose rate distribution histograms, cDRDHj, are defined by a corresponding area comprised between, on the one hand, a minimum calculated dose distribution histogram, cDDHjO, or a minimum calculated dose rate distribution histogram, cDRDHjO, and, on the other hand, a maximum calculated dose distribution histogram, cDDHj1, or a maximum calculated dose rate distribution histogram, cDRDHj1, wherein
• the minimum calculated dose distribution histogram cDDHjO or the minimum calculated dose rate distribution histogram cDRDHj0 is defined by the lowest values of the calculated dose distribution histogram cDDHj or the calculated dose rate distribution histogram cDRDHj calculated with the predefined confidence level CLj from the N randomly selected values of the monitor unit MUij, the position Xij of the beamlets, and the starting time t0ij and end time t1ij, and wherein
• the maximum calculated dose distribution histogram cDDHjO or the maximum calculated dose rate distribution histogram cDRDHjO is defined by the highest values of the calculated dose distribution histogram cDDHj or the calculated dose rate distribution histogram cDRDHj calculated with the predefined confidence level CLj from the N randomly selected values of the monitor unit MUij, the position X0i of the beamlets, and the starting time t0ij and end time t1ij.

11. Method according to any one of the preceding claims, wherein the treatment plan includes depositing doses into at least a portion of the structure of interest at ultra-high deposition rates defined as a deposition rate greater than or equal to 1 Gy / s.

12. Method according to any one of the preceding claims, wherein the planned parameters also comprise a planned beam current, Ij, whose values lij used for calculating the calculated dose distribution histogram cDDHj or dose rate distribution histogram cDRDHj are randomly selected within a corresponding tentative statistical distribution Tj of the beam current Ij.

13. Method according to any one of the preceding claims, wherein a dose distribution histogram, selected from the target dose distribution histogram or the calculated dose distribution histogram, DDH a dose volume histogram, DVH, and wherein the dose rate distribution histogram, DRDH, selected from the target dose rate distribution histogram or the calculated dose rate distribution histogram, is a dose rate volume histogram, DRVH, or a differential dose rate histogram, DDRH.

14. Error predicting module configured for implementing the method of any one of the preceding claims, comprising,
• a memory comprising the plurality of tentative statistical distributions, Tj, for each operating parameter centred on the plurality of corresponding average values, µj,
• a user interface configured for,
∘ entering the treatment plan (TP) including the one or more target dose distribution histograms, tDHDH, or target dose rate distribution histograms, tDHRDH, as well as the corresponding acceptable bands of variation, BV,
∘ selecting from the memory or entering the planned starting time, t0pi, and the planned end time, t1pi, at which each beamlet is to be delivered,
∘ selecting from the memory or entering for each beamlet, the first tentative statistical distribution, Tj, for each operating parameter, including the monitor unit, MUj, the position, Xj, of the beamlet, and the starting time, t0j, and end time, t1i, and
∘ entering the confidence level, CLj, (OLj) on the operational parameters,
• a processor configured for,
(i) randomly selecting the value MUij of the monitor unit, the value Xij of the position of the beamlet, the values of the starting time, t0ij, and end time, t1ij, comprised within the predefined confidence levels, CLj, of the corresponding tentative statistical distributions, Tj,
(ii) calculating a calculated one or more of the dose distribution histogram, cDDHj, and the calculated dose rate distribution histogram, cDRDHj, with the values thus randomly selected, and
(iii) repeating the last two steps the statistically representative number N of times to yield the calculated distributions, CDj, of the thus calculated dose distribution histograms, cDDHj, and dose rate distribution histograms, cDRDHj, for each beamlet.

15. Error predicting module according to the preceding claim 14, wherein the processor is further configured, in case any one of the one or more of the calculated distributions, CDj, of corresponding calculated dose distribution histograms (cDDHj) or dose rate distribution histograms (cDRDHj) are not comprised within the corresponding acceptable bands of variation with the predefined confidence level, CLj, for repeating steps (i) to (iii) of claim 14 with new tentative statistical distributions, T(j+k), of the operating parameters, until the calculated distributions, CDj, of the one or more of the calculated dose distribution histograms, cDDHj, or the dose rate distribution histograms, cDRDHj, are all comprised within the corresponding acceptable bands of variation with the predefined confidence level, CLj.

## Patentansprüche

1. Computerimplementiertes Verfahren zum Optimieren von Toleranzwerten von Betriebsparametern eines Teilchenbeschleunigungssystems, das es ermöglicht, dass ein Strahlenbündel, der aus einer Vielzahl von Teilchenteilstrahlen gebildet wird, die entlang einer Bestrahlungsachse (Z) beschleunigt werden, Dosen durch Bleistiftstrahlscannen (Pencil-Beam-Scanning (PBS)) in eine interessierende Struktur eines Patienten gemäß einem Behandlungsplan (TP) einbringt, wobei das computerimplementierte Verfahren umfasst
(a) Bereitstellen einer Eingabe, die Folgendes umfasst
o den Behandlungsplan (TP), der eine Definition einer Anordnung von Teilstrahlen, bi, umfasst, die jeweils durch geplante Parameter definiert sind, einschließlich,
• einer geplanten Position, Xpi, jedes Teilstrahls, bi, auf einer Ebene (X, Y) normal zur Bestrahlungsachse (Z),
• einer geplanten Monitoreinheit, MUpi, jedes Teilstrahls,
• einer geplanten Teilstrahlen-Scansequenz über die geplanten Positionen, Xpi,
∘ einen geplanten Startzeitpunkt, t0pi, und Endzeitpunkt, t1pi, zu dem jeder Teilstrahl abgegeben werden soll,
∘ eine Definition der interessierenden Struktur, die ein oder mehrere Gewebe definiert, die von einer Anzahl von Teilstrahlen, bi, durchquert werden
∘ Werte eines oder mehrerer Zieldosisverteilungshistogramme, tDDH, oder Dosisratenverteilungshistogramme, tDRDH, für die interessierende Struktur, die bei einer Behandlung mit den geplanten Parametern erhalten werden, und
∘ akzeptable Schwankungsbreiten, BV, innerhalb derer das eine oder die mehreren Zieldosisverteilungshistogramme oder Zieldosisratenverteilungshistogramme schwanken dürfen,
(b) Bereitstellen einer vorläufigen statistischen Verteilung, Tj, von Betriebsparametern des Teilchenbeschleunigungssystems, die auf entsprechende Mittelwerte, µj, zentriert ist, die für die Leistung des Teilchenbeschleunigungssystems repräsentativ sind, und Definieren eines Konfidenzniveaus, CLj, der vorläufigen statistischen Verteilung, Tj, wobei die Betriebsparameter Folgendes umfassen
∘ die Monitoreinheit, MUj, jedes Teilstrahls,
∘ die Position jedes Teilstrahls, Xj, und
∘ den Startzeitpunkt, t0j, und den Endzeitpunkt, t1j, der Abgabe jedes Teilstrahls,
(c) zufälliges Auswählen aus den entsprechenden vorläufigen statistischen Verteilungen, Tj, innerhalb der vordefinierten Konfidenzniveaus, CLj, eines Wertes MUij der Monitoreinheit, eines Wertes Xij der Position des Teilstrahls und eines Wertes t0ij, tlij jeweils des Startzeitpunktes, t0ij, und des Endzeitpunktes tlij,
(d) Berechnen eines oder mehrerer berechneter Dosisverteilungshistogramme, cDDHj, Durchlauf x, oder Dosisratenverteilungshistogramme, cDRDHj, Durchlauf x, wobei x = 1 bis N eine Anzahl von Berechnungsdurchläufen ist, mit den auf diese Weise zufällig ausgewählten Werten,
(e) Wiederholen der letzten beiden Schritte (c), (d) eine statistisch repräsentative Anzahl N von Malen, um berechnete Verteilungen, CDj, zu erhalten, die das eine oder die mehreren so berechneten Dosisverteilungshistogramme, cDDHj, oder Dosisratenverteilungshistogramme, cDRDHj, für alle auf diese Weise zufällig ausgewählten Werte der Betriebsparameter definieren,
(f) Vergleichen der berechneten Verteilungen, CDj, des einen oder der mehreren berechneten Dosisverteilungshistogramme, cDDHj, oder Dosisratenverteilungshistogramme, cDRDHj, mit den entsprechenden akzeptablen Schwankungsbreiten, BV, und Bestimmen, ob die berechneten Verteilungen, CDj, des einen oder der mehreren berechneten Dosisverteilungshistogramme, cDDHj, oder Dosisratenverteilungshistogramme, cDRDHj, in den entsprechenden akzeptablen Schwankungsbreiten innerhalb des vordefinierten Konfidenzniveaus, CLj, enthalten sind.

2. Verfahren nach Anspruch 1, umfassend Festlegen einer endgültigen statistischen Verteilung, Tf, mit einem endgültigen Konfidenzniveau, CLf, für die entsprechenden Betriebsparameter wie folgt,
• wenn die berechneten Verteilungen, CDj, des einen oder der mehreren berechneten Dosisverteilungshistogramme, cDDHj, oder Dosisratenverteilungshistogramme, cDRDHj, alle innerhalb der entsprechenden akzeptablen Schwankungsbreiten, BV, mit dem vordefinierten Konfidenzniveau, CLj, liegen, für den gegebenen Behandlungsplan (TP), Festlegen der vorläufigen statistischen Verteilung, Tj, als die endgültige statistische Verteilung Tf = Tj, und des Konfidenzniveaus, CLj, als das entsprechende endgültige Konfidenzniveau CLf = CLj, um den entsprechenden Betriebsparameter zu definieren,
• wenn eines des einen oder der mehreren berechneten Dosisverteilungshistogramme, cDDHj, oder Dosisratenverteilungshistogramme, cDRDHj, die mit einem Satz zufällig ausgewählter Werte innerhalb der entsprechenden Konfidenzniveaus, CLj, der statistischen Verteilung, Tj, der Betriebsparameter berechnet wurden, über die entsprechenden akzeptablen Schwankungsbreiten, BV, hinausgeht
∘ Wiederholen der Schritte (b) bis (f) von Anspruch 1 mit neuen vorläufigen statistischen Verteilungen, T(j+k), der Betriebsparameter und/oder
∘ Auswählen neuer, weniger ehrgeiziger Konfidenzniveaus, CL(j+k), bis die berechneten Verteilungen, CD(j+k), des einen oder der mehreren berechneten Dosisverteilungshistogramme, cDDH(j+k), oder Dosisratenverteilungshistogramme, cDRDH(j+k), alle innerhalb der entsprechenden akzeptablen Schwankungsbreiten, BV, liegen, und Festlegen der vorläufigen statistischen Verteilung, T(j+k), als endgültige statistische Verteilung Tf = T(j+k) und Festlegen des entsprechenden Konfidenzniveaus, CL(j+k), als endgültiges Konfidenzniveau CU = CL(j+k), um die entsprechenden Betriebsparameter zu definieren.

3. Verfahren nach Anspruch 2, wobei das Festlegen der endgültigen statistischen Verteilung, Tf, für die entsprechenden Betriebsparameter von einem menschlichen Bediener oder einem Prozessor durchgeführt wird.

4. Verfahren nach Anspruch 2 oder 3, wobei die neuen vorläufigen statistischen Verteilungen T(j+k), die in Anspruch 2 definiert werden, für den Fall, dass eines des einen oder der mehreren berechneten Dosisverteilungshistogramme, cDDHj, oder Dosisratenverteilungshistogramme, cDRDHj, über die entsprechenden akzeptablen Schwankungsbreiten mit dem vordefinierten Konfidenzniveau, CLj, hinausgeht, geringere Standardabweichungen, σj, aufweisen als die entsprechenden vorläufigen statistischen Verteilungen, Tj, die in Schritt (b) von Anspruch 1 definiert werden.

5. Verfahren nach einem der vorhergehenden Ansprüche, wobei die vorläufigen statistischen Verteilungen, Tj, jedes der Betriebsparameter Gaußverteilungen sind, und wobei der Wert des Konfidenzniveaus, CLj, zwischen 68% und 99,7%, bevorzugt zwischen 95,5 und 99% der vorläufigen statistischen Verteilung, liegt, wobei ein Konfidenzniveau von 68% µj ± σj entspricht, ein Konfidenzniveau von 95% µj ± 2σj entspricht und ein Konfidenzniveau von 99,7% der vorläufigen statistischen Verteilung µj ± 3σj entspricht, wobei µj ein Mittelwert und σj die Standardabweichung der entsprechenden vorläufigen statistischen Verteilungen ist.

6. Verfahren nach Anspruch 5, wobei die Mittelwerte µj und Standardabweichungen σj jedes Betriebsparameters für jeden Teilstrahl bi unterschiedlich sein dürfen.

7. Verfahren nach einem der vorhergehenden Ansprüche, wobei das Teilchenbeschleunigungssystem mit einem zyklischen Prüfmodul ausgestattet ist, das so konfiguriert ist, dass es in verschiedenen Intervallen oder kontinuierlich Ist-Werte der Betriebsparameter misst, einschließlich einer Ist-Monitoreinheit MUai, einer Ist-Position Xai und eines Ist-Wertes eines Startzeitpunktes t0ai und eines Endzeitpunktes tlai der von dem Teilchenbeschleunigungssystem emittierten Teilstrahlen.

8. Verfahren nach dem vorhergehenden Anspruch 7, wobei das Teilchenbeschleunigungssystem mit einem Prozessor ausgestattet ist, der so konfiguriert ist, dass er die Ist-Werte der Betriebsparameter mit dem entsprechenden Konfidenzniveau CLj vergleicht und eine Behandlungssitzung abbricht, falls ein Ist-Wert eines Betriebsparameters aus dem entsprechenden endgültigen Konfidenzniveau CLf herausfällt.

9. Verfahren nach einem der vorhergehenden Ansprüche, wobei die geplanten Parameter zudem eine geplante Teilstrahlgröße dj umfassen, deren Werte dij, die zur Berechnung des einen oder der mehreren berechneten Dosisverteilungshistogramme cDDHj oder Dosisratenverteilungshistogramme cDRDHj verwendet werden, zufällig innerhalb der entsprechenden vorläufigen statistischen Verteilung Tj der Teilstrahlgröße dj ausgewählt werden.

10. Verfahren nach einem der vorhergehenden Ansprüche, wobei die berechneten Verteilungen CDj des einen oder der mehreren berechneten Dosisverteilungshistogramme, cDDHj, oder Dosisratenverteilungshistogramme, cDRDHj, durch einen entsprechenden Bereich definiert sind, der zwischen einerseits, einem minimalen berechneten Dosisverteilungshistogramm, cDDHjO, oder einem minimalen berechneten Dosisratenverteilungshistogramm, cDRDHjO, und, andererseits, einem maximalen berechneten Dosisverteilungshistogramm, cDDHj1, oder einem maximalen berechneten Dosisratenverteilungshistogramm, cDRDHj1, liegt, wobei
• das minimale berechnete Dosisverteilungshistogramm cDDHj0 oder das minimale berechnete Dosisratenverteilungshistogramm cDRDHj0 durch die niedrigsten Werte des berechneten Dosisverteilungshistogramms cDDHj oder des berechneten Dosisratenverteilungshistogramms cDRDHj definiert ist, die mit dem vordefinierten Konfidenzniveau CLj aus den N zufällig ausgewählten Werten der Monitoreinheit MUij, der Position Xij der Teilstrahlen und dem Startzeitpunkt t0ij und Endzeitpunkt tlij berechnet werden, und
und wobei
• das maximale berechnete Dosisverteilungshistogramm cDDHj0 oder das maximale berechnete Dosisratenverteilungshistogramm cDRDHj0 durch die höchsten Werte des berechneten Dosisverteilungshistogramms cDDHj oder des berechneten Dosisratenverteilungshistogramms cDRDHj definiert ist, die mit dem vordefinierten Konfidenzniveau CLj aus den N zufällig ausgewählten Werten der Monitoreinheit MUij, der Position X0i der Teilstrahlen und dem Startzeitpunkt t0ij und dem Endzeitpunkt tlij berechnet werden.

11. Verfahren nach einem der vorhergehenden Ansprüche, wobei der Behandlungsplan Einbringen von Dosen in zumindest einen Teil der interessierenden Struktur mit ultrahohen Einbringungsraten, definiert als eine Einbringungsrate von mehr als oder gleich 1 Gy/s, umfasst.

12. Verfahren nach einem der vorhergehenden Ansprüche, wobei die geplanten Parameter zudem einen geplanten Strahlstrom lj umfassen, dessen Werte lij, die zum Berechnen des berechneten Dosisverteilungshistogramms cDDHj oder Dosisratenverteilungshistogramms cDRDHj verwendet werden, zufällig innerhalb einer entsprechenden vorläufigen statistischen Verteilung Tj des Strahlstroms lj ausgewählt werden.

13. Verfahren nach einem der vorhergehenden Ansprüche, wobei ein Dosisverteilungshistogramm, ausgewählt aus dem Zieldosisverteilungshistogramm oder dem berechneten Dosisverteilungshistogramm, DDH, ein Dosisvolumenhistogramm, DVH, ist und wobei das Dosisratenverteilungshistogramm, DRDH, ausgewählt aus dem Zieldosisratenverteilungshistogramm oder dem berechneten Dosisratenverteilungshistogramm, ein Dosisratenvolumenhistogramm, DRVH, oder ein Differenzdosisratenhistogramm, DDRH, ist.

14. Fehlerprognosemodul, das zum Implementieren des Verfahrens nach einem der vorhergehenden Ansprüche konfiguriert ist, umfassend,
• einen Speicher, der die Vielzahl vorläufiger statistischer Verteilungen, Tj, für jeden Betriebsparameter umfasst, die auf die Vielzahl entsprechender Mittelwerte, µj, zentriert sind,
• eine Benutzerschnittstelle, die konfiguriert ist zum
∘ Eingeben des Behandlungsplans (TP) einschließlich des einen oder der mehreren Zieldosisverteilungshistogramme, tDHDH, oder Zieldosisratenverteilungshistogramme, tDHRDH, sowie der entsprechenden akzeptablen Schwankungsbreiten, BV,
∘ Auswählen aus dem Speicher oder Eingeben des geplanten Startzeitpunktes, t0pi, und des geplanten Endzeitpunktes, t1pi, zu dem jeder Teilstrahl abgegeben werden soll,
∘ Auswählen aus dem Speicher oder Eingeben für jeden Teilstrahl der ersten vorläufigen statistischen Verteilung, Tj, für jeden Betriebsparameter, einschließlich der Monitoreinheit, MUj, der Position, Xj, des Teilstrahls sowie des Startzeitpunktes, t0j, und des Endzeitpunktes, tli, und
∘ Eingeben des Konfidenzniveaus, CLj, für die Betriebsparameter,
• einen Prozessor, der konfiguriert ist zum
(i) zufälligen Auswählen des Wertes MUij der Monitoreinheit, des Wertes Xij der Position des Teilstrahls, der Werte des Startzeitpunktes, t0ij, und des Endzeitpunktes, tlij, die innerhalb der vordefinierten Konfidenzniveaus, CLj, der entsprechenden vorläufigen statistischen Verteilungen, Tj, liegen,
(ii) Berechnen eines oder mehrerer berechneter Dosisverteilungshistogramme, cDDHj, und des berechneten Dosisratenverteilungshistogramms, cDRDHj, mit den auf diese Weise zufällig ausgewählten Werten, und
(iii) Wiederholen der letzten beiden Schritte eine statistisch repräsentative Anzahl N von Malen, um die berechneten Verteilungen, CDj, der auf diese Weise berechneten Dosisverteilungshistogramme, cDDHj, und Dosisratenverteilungshistogramme, cDRDHj, für jeden Teilstrahl zu erhalten.

15. Fehlerprognosemodul nach dem vorhergehenden Anspruch 14, wobei der Prozessor ferner so konfiguriert ist, dass er für den Fall, dass eine oder mehrere der einen oder der mehreren der berechneten Verteilungen, CDj, entsprechendener berechneter Dosisverteilungshistogramme (cDDHj) oder Dosisratenverteilungshistogramme (cDRDHj) nicht innerhalb der entsprechenden akzeptablen Schwankungsbreiten mit dem vordefinierten Konfidenzniveau, CLj, liegen, die Schritte (i) bis (iii) von Anspruch 14 mit neuen vorläufigen statistischen Verteilungen T(j+k) der Betriebsparameter wiederholt, bis die berechneten Verteilungen, CDj, des einen oder der mehreren berechneten Dosisverteilungshistogramme, cDDHj, oder Dosisratenverteilungshistogramme, cDRDHj, alle innerhalb der entsprechenden akzeptablen Schwankungsbreiten mit dem vordefinierten Konfidenzniveau, CLj, liegen.

## Revendications

1. Procédé informatisé pour optimiser des valeurs de tolérance de paramètres de fonctionnement d'un système d'accélération de particules permettant à un faisceau, formé par une pluralité de petits faisceaux de particules accélérées le long d'un axe d'irradiation (Z), de déposer des doses par la méthode dite « pencil beam scanning » (= PBS) dans une structure d'intérêt d'un patient selon un plan de traitement (TP), ce procédé informatisé comprenant :
(a) la fourniture de données d'entrée comprenant :
o le plan de traitement (TP) comprenant une définition d'un réseau de petits faisceaux, bi, chacun étant défini par des paramètres selon plan, comprenant :
• une position selon plan, Xpi, de chaque petit faisceau, bi, sur un plan (X, Y) normal à l'axe d'irradiation (Z),
• une unité de surveillance selon plan, MUpi, de chaque petit faisceau,
• une séquence de balayage par petit faisceau selon plan sur les positions selon plan, Xpi,
∘ une heure de début, t0pi et une heure de fin, T1pi, selon plan auxquelles chaque petit faisceau doit être fourni,
o une définition de la structure d'intérêt, définissant un ou plusieurs tissus étant traversés par un certain nombre des petits faisceaux, bi,
∘ des valeurs d'un ou de plusieurs histogrammes de distribution de doses, tDDH, ou d'histogrammes de distribution de débits de doses, tDRDH, cibles pour la structure d'intérêt, obtenues avec un traitement avec les paramètres selon plan, et
∘ des bandes de variation acceptables, BV, à l'intérieur desquelles le ou les histogrammes de distribution de doses ou histogrammes de distribution de débits de doses cibles sont autorisés à varier,
(b) la fourniture d'une distribution statistique provisoire, Tj, des paramètres de fonctionnement du système d'accélération de particules centré sur des valeurs moyennes correspondantes, µj, représentatives de la performance du système d'accélération de particules et la définition d'un niveau de confiance, CLj, de la distribution statistique provisoire, Tj, ces paramètres de fonctionnement comprenant :
o l'unité de surveillance, MUj, de chaque petit faisceau,
o la position de chaque petit faisceau, Xj, et
o l'heure de début, t0j, et l'heure de fin, T1j, de la fourniture de chaque petit faisceau,
(c) la sélection aléatoire, à partir de la distribution statistique provisoire correspondante, Tj, au sein des niveaux de confiance prédéfinis, CLj, d'une valeur MUij de l'unité de surveillance, d'une valeur Xij de la position du petit faisceau, et d'une valeur t0ij, tlij, de chacune de l'heure de début, T0ij, et de l'heure de fin, tlij,
(d) le calcul d'un ou de plusieurs histogrammes de distribution de doses calculés, CDDHj, cycle x, ou d'histogrammes de distribution de débits de doses, cDRDHj, cycle x, avec x = 1 à N, N étant un nombre de cycles de calcul, avec les valeurs sélectionnées ainsi de manière aléatoire,
(e) la répétition des deux dernières étapes (c), (d), un nombre N de fois statistiquement représentatif de façon à obtenir des distributions calculées, CDj, définissant le ou les histogrammes de distribution de doses, cDDHj, ou histogrammes de distribution de débits de doses, cDRDHj, calculés pour toutes les valeurs des paramètres de fonctionnement sélectionnées ainsi de manière aléatoire,
(f) la comparaison des distributions calculées, CDj, du ou des histogrammes de distribution de doses, cDDHj, ou histogrammes de distribution de débits de doses, cDRDHj, calculés avec les bandes de variation acceptables correspondantes, BV, et la détermination de si les distributions calculées, CDj, du ou des histogrammes de distribution de doses, cDDHj, ou histogrammes de distribution de débits de doses, cDRDHj, calculés sont comprises ou pas dans les bandes de variation acceptables correspondantes au sein du niveau de confiance prédéfini, CLj .

2. Procédé selon la revendication 1, comprenant l'établissement d'une distribution statistique finale, Tf, avec un niveau de confiance final, pour définir les paramètres de fonctionnement correspondants en procédant de la manière suivante :
• si les distributions calculées, CDj, du ou des histogrammes de distribution de doses, cDDHj, ou histogrammes de distribution de débits de doses, cDRDRHj, calculés sont tous compris à l'intérieur des bandes de variation acceptables, BV, correspondantes, avec le niveau de confiance prédéfini, CLj, pour le plan de traitement (TP) donné, l'établissement de la distribution statistique provisoire, Tj, comme la distribution statistique finale, Tf = Tj, et le niveau de confiance, CLj comme le niveau de confiance final correspondant, CLf = CLj, afin de définir le paramètre de fonctionnement correspondant,
• si un quelconque du ou des histogrammes de distribution de doses, cDDHj, ou histogrammes de distribution de débits de doses, cDRDRHj, calculés, calculés avec un ensemble de valeurs sélectionnées de manière aléatoire au sein des niveaux de confiance correspondants, CLj, de la distribution statistique, Tj, des paramètres de fonctionnement s'étend au-delà des bandes de variation acceptables, BV, correspondantes,
∘ la répétition des étapes (b) à (f) de la revendication 1 avec de nouvelles distributions statistiques provisoires, T(j + k), des paramètres de fonctionnement, et/ou
∘ la sélection de nouveaux niveaux de confiance moins ambitieux, CL(j + k), jusqu'à ce que les distributions calculées, CD(j + k), du ou des histogrammes de distribution de doses, cDDH(j + k), ou histogrammes de distribution de débits de doses, cDRDRH(j + k), calculés sont tous compris à l'intérieur des bandes de variation acceptables, BV, correspondantes, et l'établissement de la distribution statistique provisoire, T(j + k), comme la distribution statistique finale, Tf = T(j + k), et l'établissement du niveau de confiance correspondant, CL(j + k), comme le niveau de confiance final, CLf = CL(j + k), afin de définir les paramètres de fonctionnement correspondants.

3. Procédé selon la revendication 2, dans lequel l'établissement de la distribution statistique finale, Tf, pour définir les paramètres de fonctionnement correspondants est exécuté par un opérateur humain ou par un processeur.

4. Procédé selon la revendication 2 ou 3, dans lequel les nouvelles distributions statistiques provisoires, T(j + k), définies dans la revendication 2 au cas où un quelconque du ou des histogrammes de distribution de doses, cDDHj, ou histogrammes de distribution de débits de doses, cDRDRHj, calculés s'étend au-delà des bandes de variation acceptables correspondantes avec le niveau de confiance prédéfini, CLj, a des écarts types σj plus bas que les distributions statistiques provisoires correspondantes, Tj, définies lors de l'étape (b) de la revendication 1.

5. Procédé selon l'une quelconque des revendications précédentes, dans lequel les distributions statistiques provisoires correspondantes, Tj, de chacun des paramètres de fonctionnement sont des distributions gaussiennes, et dans lequel la valeur du niveau de confiance, CLj, est comprise entre 6 8 % et 99,7 %, de préférence entre 95,5 et 99 % de la distribution statistique provisoire, un niveau de confiance de 68 % correspondant µj ± σj, un niveau de confiance de 95 % correspondant à µj ± 2 σj, et un niveau de confiance de 99,7 % de la distribution statistique provisoire correspondant à µj ± 3 σj, µj étant une valeur moyenne et σj étant l'écart type des distributions statistiques provisoires correspondantes.

6. Procédé selon la revendication 5, dans lequel les valeurs moyennes µj et les écarts types σj de chaque paramètre de fonctionnement sont autorisés à être différents pour chaque petit faisceau, bi.

7. Procédé selon l'une quelconque des revendications précédentes, dans lequel le système d'accélération de particules est équipé d'un module de vérification cyclique pour mesurer à des intervalles différents ou continuellement des valeurs réelles des paramètres de fonctionnement comprenant une unité de surveillance réelle, MUai, une position réelle, Xai, et une valeur réelle d'une heure de début, T0ai et d'une heure de fin, tlai, des petits faisceaux émis par le système d'accélération de particules.

8. Procédé selon la revendication précédente 7, dans lequel le système d'accélération de particules est équipé d'un processeur configuré de façon à comparer les valeurs réelles des paramètres de fonctionnement avec le niveau de confiance correspondant, CLj, et à arrêter une session de traitement au cas où une valeur réelle d'un paramètre de fonctionnement se situe hors du niveau de confiance final correspondant, CLf.

9. Procédé selon l'une quelconque des revendications précédentes, dans lequel les paramètres selon plan comprennent aussi une taille selon plan de petit faisceau, dj, dont les valeurs, dij, utilisées pour calculer le ou les histogrammes de distribution de doses, cDDHj, ou histogrammes de distribution de débits de doses, cDRDHj, calculés sont sélectionnés de manière aléatoire au sein de la distribution statistique provisoire, tj, correspondante de la taille de petit faisceau, dj.

10. Procédé selon l'une quelconque des revendications précédentes, dans lequel les distributions calculées, CDj, du ou des histogrammes de distribution de doses, cDDHj, ou histogrammes de distribution de débits de doses, cDRDHj, calculés sont définies par une aire correspondante comprise entre, d'une part, un histogramme de distribution de doses calculé minimum, cDDHjO, ou un histogramme de débit de doses calculé minimum, cDRDHjO, et, d'autre part, un histogramme de distribution de doses calculé maximum, cDDHj1, ou un histogramme de distribution de débits de doses calculé maximum, cDRDHj1, dans lequel
• l'histogramme de distribution de dose calculé minimum, cDDHjO, ou l'histogramme de débit de dose calculé minimum, cDRDHjO, est défini par les valeurs les plus basses de l'histogramme de distribution de dose calculé, cDDHj, ou de l'histogramme de débit de dose calculé, cDRDHjO, calculés avec le niveau de confiance prédéfini, CLj, à partir des N valeurs sélectionnées de manière aléatoire de l'unité de surveillance, MUij, de la position, Xij, des petits faisceaux, et de l'heure de début, T0ij et de l'heure de fin, tlij, et dans lequel
• l'histogramme de distribution de dose calculé maximum, cDDHjO, ou l'histogramme de débit de dose calculé maximum, cDRDHjO, est défini par les valeurs les plus hautes de l'histogramme de distribution de doses calculé, cDDHj, ou de l'histogramme de débit de doses calculé, cDRDHj, calculés avec le niveau de confiance prédéfini, CLj, à partir des N valeurs sélectionnées de manière aléatoire de l'unité de surveillance, MUij, de la position, X0i, des petits faisceaux, et de l'heure de début, t0ij et de l'heure de fin, tlij.

11. Procédé selon l'une quelconque des revendications précédentes, dans lequel le plan de traitement comprend le dépôt de doses dans au moins une partie de la structure d'intérêt à des vitesses de dépôt extrêmement élevées définies comme une vitesse de dépôt plus grande que ou égale à 1 Gy / s.

12. Procédé selon l'une quelconque des revendications précédentes, dans lequel les paramètres selon plan comprennent aussi un courant de faisceau selon plan, Ij, dont les valeurs Iij pour calculer l'histogramme de distribution de doses, cDDHi, ou l'histogramme de distribution de débit de doses, cDFDHj, calculés sont sélectionnées de manière aléatoire au sein d'une distribution statistique provisoire correspondante, tj, du courant de faisceau, Ij.

13. Procédé selon l'une quelconque des revendications précédentes, dans lequel un histogramme de distribution de doses, sélectionné parmi l'histogramme de distribution de doses cible ou l'histogramme de distribution de doses calculé, DDH, est un histogramme de volume de doses, DVH, et dans lequel l'histogramme de distribution de débit de doses, DRDH, sélectionné parmi l'histogramme de distribution de débit de doses cible ou l'histogramme de distribution de débit de doses calculé est un histogramme de volume de débit de doses, DRVH, ou un histogramme de débit de doses différentiel, DDRH.

14. Module de prédiction d'erreurs configuré de façon à exécuter le procédé selon l'une quelconque des revendications précédentes, comprenant :
• une mémoire comprenant la pluralité de distributions statistiques provisoires, Tj, pour chaque paramètre de fonctionnement centré sur la pluralité de valeurs moyennes correspondantes,
• une interface utilisateur configurée de façon à
∘ entrer le plan de traitement (TP) comprenant le ou les histogrammes de distribution de doses, tDHDH, ou histogrammes de distribution de débits de doses, tDHRDH, cibles de même que les bandes de variation acceptables correspondantes, BV,
o sélectionner dans la mémoire ou entrer l'heure de début selon plan, t0pi, et l'heure de fin selon plan, t1pi, auxquelles chaque petit faisceau doit être fourni,
∘ sélectionner dans la mémoire ou entrer, pour chaque petit faisceau, la première distribution statistique provisoire, Tj, pour chaque paramètre de fonctionnement, comprenant l'unité de surveillance, MUj, la position, Xj, du petit faisceau, et l'heure de début, t0j, et l'heure de fin, t1j ; et à
o entrer le niveau de confiance, CLj, sur les paramètres de fonctionnement,
• un processeur configuré de façon à :
(i) sélectionner de manière aléatoire la valeur MUij de l'unité de surveillance, la valeur Xij de la position du petit faisceau, les valeurs de l'heure de début, t0ij, et de l'heure de fin, tlij, comprises au sein des niveaux de confiance prédéfinis, CLj, des distributions statistiques provisoires, Tj, correspondantes,
(ii) calculer un ou plusieurs des histogrammes de distribution de doses, cDDHj, et des histogrammes de distribution de débits de doses, cDRDHj, calculés avec les valeurs sélectionnées ainsi de manière aléatoire, et à
(iii) répéter les deux dernières étapes le nombre N de fois statistiquement représentatif afin d'obtenir les distributions calculées, CDj, des histogrammes de distribution de doses, cDDHj, et des histogrammes de distribution de débits de doses, cDRDHj, ainsi calculés pour chaque petit faisceau.

15. Module de prédiction d'erreurs selon la revendication précédente 14, dans lequel le processeur est configuré en outre de façon à, au cas où une quelconque de la ou des distributions calculées, CDj, des histogrammes de distribution de doses (cDDHj) ou des histogrammes de distribution de débits de doses (cDHDRj) calculés correspondants n'est pas comprise à l'intérieur de bandes de variation acceptables correspondantes avec le niveau de confiance prédéfini, CLj, répéter les étapes (i) à (iii) de la revendication 14 avec de nouvelles distributions statistiques provisoires, T(j + k), des paramètres de fonctionnement, jusqu'à ce que les distributions calculées, CDj, du ou des histogrammes de distribution de doses, cDDHj, ou des histogrammes de distribution de débits de doses, cDHDRj, calculés soient toutes comprises à l'intérieur des bandes de variation acceptables correspondantes avec le niveau de confiance prédéfini, CLj.
